# EUROPEAN PATENT APPLICATION

(11) **EP 3 879 538 A1**
(43) Date of publication of application: **15.09.2021**
(21) Application number: 21153422.7
(22) Date of filing: 26.01.2021
(51) Int. Cl.: G16H 10/20, G16H 50/70

(54) **INFORMATION PROCESSING METHOD, INFORMATION PROCESSING PROGRAM, AND INFORMATION PROCESSING DEVICE**

(30) Priority: 13.03.2020 JP 2020044410
(71) Applicant: FUJITSU LIMITED, Kanagawa 211-8588 (JP)
(72) Inventor: Oya, Takuro, Kanagawa, 211-8588 (JP); Aki, Michihiko, Kanagawa, 211-8588 (JP)
(74) Representative: Hoffmann Eitle

(57) **Abstract**

An information processing method, includes: dividing by a computer, for each of a plurality of factors common in clinical trial information of a first patient group and real-world data of second patient group, each of the first patient group and the second patient group into new patient groups by a predetermined value; and generating information regarding a candidate of a factor related to new efficacy of an existing drug for each of the plurality of factors based on a behavior of a predetermined patient group related to the second patient group among the new patient groups and behaviors of other patient groups among the new patient groups.

## Description

### [Technical Field]

The embodiment discussed herein is related to an information processing method, an information processing program, and an information processing device.

### [Background Art]

In recent years, pharmaceutical companies have conducted development of drugs using real-world data obtained in clinical sites such as electronic medical records and patient diaries. Examples thereof include drug repositioning, that is, finding new efficacy of an existing drug from the real-world data and developing the drug as a therapeutic drug for a different disease.

As a method of implementing the repositioning, for example, a method has been proposed of finding a factor related to new efficacy that is not found in a past clinical trial, conducting a clinical trial for patients classified using this factor, and evaluating whether the new efficacy is obtained.
[PTL 1] International Publication Pamphlet No. WO 2008/111349
[PTL 2] Japanese Laid-open Patent Publication No. 2007-26172

However, up to now, since the factor related to the new efficacy is searched for from the real-world data in a trial and error manner, it is difficult to find this factor.

According to an aspect, the present disclosure is aimed at assisting the drug repositioning.

### [Solution to Problem]

According to an aspect of the embodiment, an information processing method, includes: dividing by a computer, for each of a plurality of factors common in clinical trial information of a first patient group and real-world data of second patient group, each of the first patient group and the second patient group into new patient groups by a predetermined value; and generating information regarding a candidate of a factor related to new efficacy of an existing drug for each of the plurality of factors based on a behavior of a predetermined patient group related to the second patient group among the new patient groups and behaviors of other patient groups among the new patient groups.

### [Effects of Invention]

According to an aspect of the embodiments, the drug repositioning may be assisted.

### [Brief Description of Drawings]

FIG. 1 is a diagram illustrating an example of a system configuration of an information processing system.
FIG. 2 is a diagram for describing processing for identifying a factor with which there is a possibility that new efficacy is obtained by an existing drug.
FIG. 3 is a diagram illustrating an example of a hardware configuration of an information processing device.
FIG. 4 illustrates examples of clinical trial information and real-world data.
FIG. 5 is a diagram for describing a functional configuration of the information processing device.
FIG. 6 is a first flowchart describing processing of the information processing device.
FIG. 7 is a second flowchart describing the processing of the information processing device.
FIG. 8 is a third flowchart describing the processing of the information processing device.
FIG. 9 is a diagram for describing setting of a threshold for a common factor.
FIG. 10 is a diagram for describing an objective function.
FIG. 11 is a diagram for describing a difference of objective fu nctions.
FIG. 12 is a diagram for describing resetting of the threshold for the common factor.
FIG. 13 is a first diagram illustrating a display example.
FIG. 14 is a second diagram illustrating a display example.
FIG. 15 is a third diagram illustrating a display example.

### [Description of Embodiment]

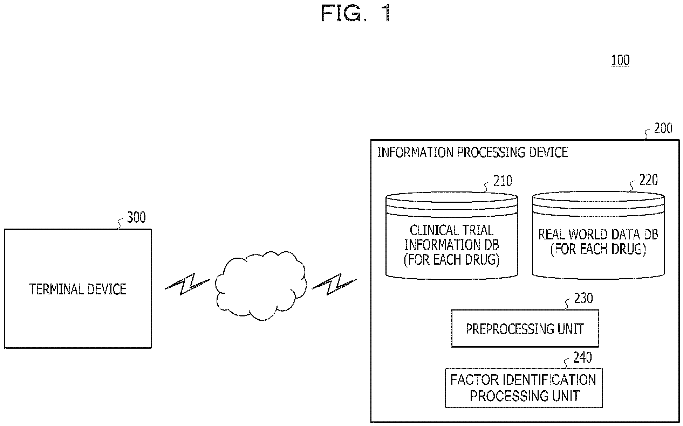

Hereinafter, an embodiment will be described with reference to the drawings. FIG. 1 is a diagram illustrating an example of a system configuration of an information processing system.

An information processing system 100 of the present embodiment includes an information processing device 200 and a terminal device 300. The information processing device 200 is coupled to the terminal device 300 via a network such as the Internet, for example.

The information processing device 200 of the present embodiment has clinical trial information database (DB) 210, real-world data database (DB) 220, a preprocessing unit 230, and a factor identification processing unit 240.

The clinical trial information DB 210 of the present embodiment stores clinical trial information indicating a result of a past clinical trial conducted for a patient group before approval of an existing drug. The real-world data DB 220 stores real-world data of a patient group having received a therapy using the existing drug. The real-world data is data in unit of anonymous patient, which is obtained from clinical sites, and is obtained from an electronic medical record system or the like introduced to a medical institution, for example.

In the following description, a patient group having received a past clinical trial conducted before the approval of the existing drug is referred to as a first patient group, and a patient group having received a therapy using this existing drug after the approval of the existing drug is referred to as a second patient group. Therefore, the clinical trial information is medical information corresponding to the first patient group, and the real-world data is medical information corresponding to the second patient group.

The preprocessing unit 230 of the present embodiment collects and stores the clinical trial information and the real-world data in the clinical trial information DB 210 and the real-world data DB 220, respectively, for each existing drug.

The factor identification processing unit 240 refers to the clinical trial information DB 210 and the real-world data DB 220 regarding an existing drug set as a repositioning target, and generates information indicating a factor with which there is a possibility that new efficacy is obtained by the existing drug. The factor identification processing unit 240 identifies a factor having a high probability that new efficacy is obtained from based on information of the factor.

The drug repositioning refers to finding of new efficacy of an existing drug already approved as a therapeutic drug for a disease, and application of the drug to a therapeutic drug for another disease. The existing drug of the present embodiment refers to a medical product already approved as a therapeutic drug for a disease.

The factor of the present embodiment is a variable for dividing the clinical trial information and the real-world data for each of patients into groups having a commonality. For example, the factor of the present embodiment is a variable for stratification of patients using a certain commonality to check efficacy of a drug.

For example, the factor of the present embodiment is a common item included in the clinical trial information and the real-world data. The items included in the clinical trial information and the real-world data include, for example, an item indicating a profile of a patient, an item indicating a vital sign of a patient, and an item indicating a clinical examination value.

The items indicating the profile of the patient include sex, age, height, weight, and race, the items indicating the vital sign of the patient include blood pressure, pulse, and body temperature, and the items indicating the clinical examination value include blood cell and urine, for example.

The terminal device 300 is a terminal device used in a pharmaceutical company, for example. In the information processing device 200 of the present embodiment, when an operation is performed to input an existing drug set as a repositioning target in the terminal device 300, the factor identification processing unit 240 executes the processing regarding this existing drug. The information processing device 200 notifies the terminal device 300 of information generated by the processing by the factor identification processing unit 240 and a candidate of an identified factor.

In this manner, according to the present embodiment, the factor with which there is a possibility that the new efficacy is obtained by the existing drug may be presented. Therefore, for example, a pharmaceutical company or the like corresponding to a user of the terminal device 300 may easily find a candidate of the factor with which there is a possibility that the new efficacy is obtained. Therefore, according to the present embodiment, the drug repositioning may be assisted.

In the example of FIG. 1, the information processing device 200 has the clinical trial information DB 210 and the real-world data DB 220 but is not limited to this. A part or all of the clinical trial information DB 210 and the real-world data DB 220 may be disposed in a device other than the information processing device 200.

The information processing device 200 has the preprocessing unit 230 and the factor identification processing unit 240 but is not limited to this. The preprocessing unit 230 and the factor identification processing unit 240 may be respectively implemented by different information processing devices.

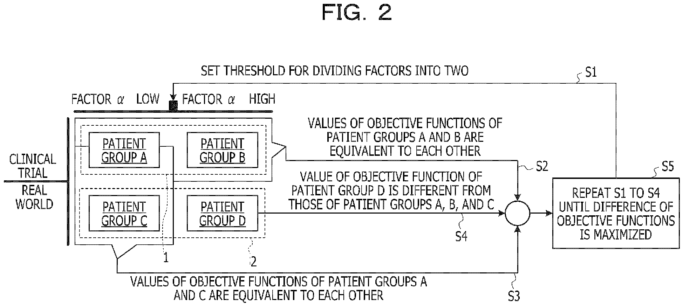

Processing of the factor identification processing unit 240 of the present embodiment is described below with reference to FIG. 2. FIG. 2 is a diagram for describing processing for identifying a factor with which there is a possibility that new efficacy is obtained by an existing drug.

The factor identification processing unit 240 of the present embodiment identifies a factor α that is common included in both the clinical trial information and the real-world data, sets a threshold for dividing values into two for the factor α, and divides each of a first patient group 1 and a second patient group 2 according to the threshold of the factor α (step S1). In the following description, the factor that is common included in both the clinical trial information and the real-world data is referred to as a common factor in some cases.

In the example of FIG. 2, the first patient group 1 is divided into a patient group A where the value of the factor α is below the threshold and a patient group B where the value of the factor α is equal to or higher than the threshold, and the second patient group 2 is divided into a patient group C where the value of the factor α is below the threshold and a patient group D where the value of the factor α is equal to or higher than the threshold. The patient groups A and B are patient groups newly divided from the first patient group 1. The patient groups C and D are patient groups newly divided from the second patient group 2.

Subsequently, the factor identification processing unit 240 performs survival time analysis based on each of clinical trial information of the patient group A and clinical trial information of the patient group B, and obtains values of objective functions (step S2).

The objective function of the present embodiment is an evaluation value obtained from a plurality of patient groups stratified by a certain factor. In the example of FIG. 2, since the clinical trial information of the patient group A and the patient group B is information obtained by the clinical trial implemented in a state where the environment is controlled, values of the objective functions are equivalent to each other.

For example, regarding the factor α, when the first patient group is divided into the patient group A and the patient group B according to the threshold for dividing the values of the factor α into two, the evaluation value obtained from the patient group A and the evaluation value obtained from the patient group B are equivalent to each other, and a behavior of the patient group A and a behavior of the patient group B are equivalent to each other.

Subsequently, the factor identification processing unit 240 obtains a value of the objective function from the real-world data of the patient group C. In the example of FIG. 2, the value of the objective function of the patient group C is equivalent to the value of the objective function of the patient group A (step S3).

For example, when the patient group C has received a therapy using this existing drug, the patient group C where the factor α is below the threshold has a behavior similar to that of the patient group A having received the clinical trial.

Next, the factor identification processing unit 240 obtains a value of the objective function from the real-world data of the patient group D (step S4). In the example of FIG. 2, the value of the objective function of the patient group D is different from the value of the objective function of the patient group B.

For example, when the patient group D has received the therapy using this existing drug, the patient group D where the factor α is equal to or higher than the threshold has a behavior different from that of the patient group B having received the clinical trial.

In this case, the real-world data of the patient group D where the factor α is equal to or higher than the threshold is not similar to the clinical trial information obtained by the clinical trial conducted for the existing drug in the past, and it is conceivable that there is a possibility that a relationship that is not found in the clinical trial exists between the factor α and the existing drug.

Next, the factor identification processing unit 240 changes and sets the threshold of the factor α again, and repeats the processing in step S1 to step S4 until a difference between the value of the objective function of the patient group B and the value of the objective function of the patient group D is maximized (step S5).

The factor identification processing unit 240 repeats the processing in step S1 to step S5 for all common factors included in the clinical trial information and the real-world data.

According to the present embodiment, as a result of the processing in step S1 to step S5 performed for all the common factors, information including the difference of the objective functions for each common factor may be output to the terminal device 300 as information indicating a factor with which there is a possibility that the new efficacy is obtained by the existing drug.

According to the present embodiment, as a result of the processing in step S1 to step S5 performed for all the common factors, a common factor having the largest difference of the objective functions may be identified, and the identified common factor may be output to the terminal device 300 as a candidate of the factor having a high probability that the new efficacy of the existing drug may be obtained.

In this manner, according to the present embodiment, when the clinical trial information and the real-world data are used in combination, information indicating the candidate of the factor related to the new efficacy of the existing drug may be generated, and this information may be provided to a pharmaceutical company or the like. Therefore, according to the present embodiment, the drug repositioning may be assisted.

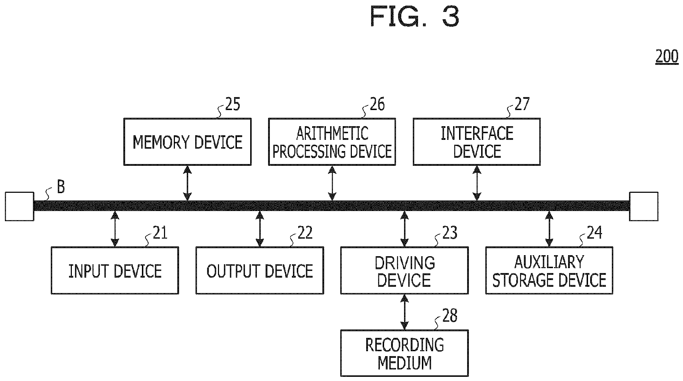

The information processing device 200 of the present embodiment is described below. FIG. 3 is a diagram illustrating an example of a hardware configuration of an information processing device.

The information processing device 200 of the present embodiment is a computer including an input device 21, an output device 22, a driving device 23, an auxiliary storage device 24, a memory device 25, an arithmetic processing device 26, and an interface device 27, which are coupled to one another via a bus B.

For example, the input device 21 is used to input various information and is implemented by a keyboard, a pointing device, or the like. For example, the output device 22 is used to output various information and is implemented by a display, or the like. Examples of the interface device 27 include a local area network (LAN) card or the like and is used for coupling to a network.

A factor identification program for implementing the factor identification processing unit 240 is at least a part of various programs for controlling the information processing device 200. For example, the factor identification program is provided through distribution of a recording medium 28, downloading from the network, or the like. As the recording medium 28 for recording the factor identification program, various types of recording media may be used, for example, a recording medium that optically, electrically, or magnetically records information, such as a compact disc read-only memory (CD-ROM), a flexible disk, or a magneto-optical disc, and a semiconductor memory that electrically records information such as a ROM or a flash memory.

When the recording medium 28 recording the factor identification program is set in the driving device 23, the factor identification program is installed in the auxiliary storage device 24 from the recording medium 28 via the driving device 23. The factor identification program downloaded from the network is installed in the auxiliary storage device 24 via the interface device 27.

The auxiliary storage device 24 implements the clinical trial information DB 210, the real-world data DB 220, and the like included in the information processing device 200, stores the factor identification program installed in the information processing device 200, and also stores various files, data, and the like used by the information processing device 200. The memory device 25 reads the factor identification program from the auxiliary storage device 24 upon the activation of the information processing device 200 and stores the read factor identification program. The arithmetic processing device 26 implements various processes described later according to the factor identification program stored in the memory device 25.

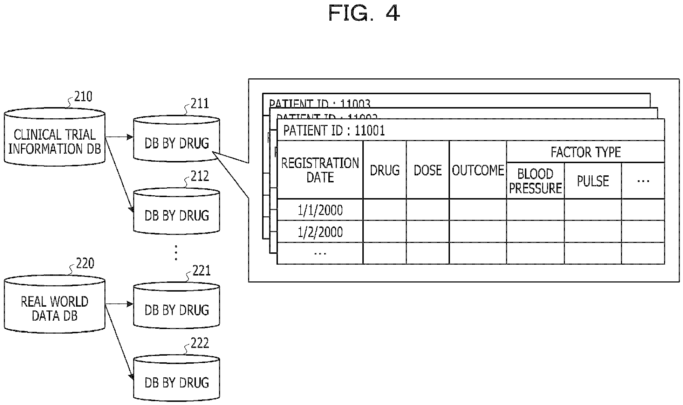

Next, the clinical trial information DB 210 and the real-world data DB 220 of the present embodiment are described with reference to FIG. 4. For example, the clinical trial information DB 210 and the real-world data DB 220 are implemented by the auxiliary storage device 24, the memory device 25, and the like. FIG. 4 illustrates examples of clinical trial information and real-world data.

The clinical trial information DB 210 of the present embodiment stores clinical trial information for each existing drug. For example, the clinical trial information DB 210 stores databases by drug 211 and 212 that store clinical trial information for each existing drug, and the like.

Similarly as in the clinical trial information DB 210, the real-world data DB 220 of the present embodiment stores real-world data for each existing drug. For example, the real-world data DB 220 stores databases by drug 221 and 222 that store real-world data for each existing drug, and the like.

The clinical trial information stored in the databases by drug 211 and 212 includes patient ID, registration date, drug, dose, outcome, factor type, and the like as items of information, and the item "patient ID" is associated with the other items.

In the following description, in the clinical trial information DB 210, information including a value of the item "patient ID" and values of the other items may be referred to as clinical trial information for each patient.

The value of the item "patient ID" indicates identification information for identifying a patient. A value of the item "registration date" indicates a date when the clinical trial information for each patient is stored in the database. A value of the item "drug" indicates a drug. A value of the item "dose" indicates a dose of the drug.

A value of the item "outcome" indicates a state of the patient. For example, the value of the item "outcome" indicates whether the patient is fully recovered, under treatment, or the like. The item "factor type" is associated with a type of the factor. For example, the item "factor type" is associated with a "blood pressure", "pulse", or the like serving as a factor.

According to the present embodiment, the real-world data stored in the real-world data DB 220 also includes items similar to those of the clinical trial information, and the description is omitted.

According to the present embodiment, the clinical trial information DB 210 and the real-world data DB 220 include databases by drug 211, 212, 221, and 222 but are not limited to this.

For example, the clinical trial information DB 210 may have database by drug and dose that stores clinical trial information for each set of the drug and the dose. The real-world data DB 220 may have database by drug and dose that stores real-world data for each set of the drug and the dose.

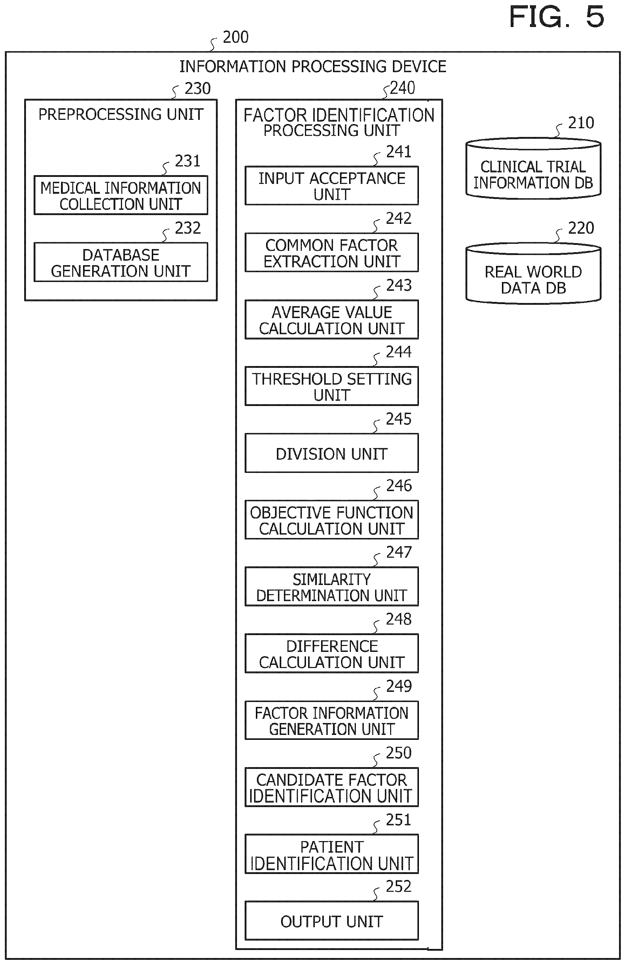

Next, a function of the information processing device 200 of the present embodiment is described with reference to FIG. 5. FIG. 5 is a diagram for describing a functional configuration of the information processing device 200.

The information processing device 200 of the present embodiment has the preprocessing unit 230 and the factor identification processing unit 240. The preprocessing unit 230 and the factor identification processing unit 240 are implemented when the arithmetic processing device 26 reads and executes the programs stored in the memory device 25 or the like.

First, the preprocessing unit 230 is described. The preprocessing unit 230 of the present embodiment performs preprocessing before processing by the factor identification processing unit 240 is executed.

The preprocessing unit 230 has a medical information collection unit 231 and a database generation unit 232. The medical information collection unit 231 collects medical information from an electronic medical record system or the like, a server device of a medical institution, or the like that communicates with the information processing device 200. The medical information includes clinical trial information and real-world data.

The database generation unit 232 classifies clinical trial information and real-world data out of the medical information collected by the medical information collection unit 231. The database generation unit 232 classifies the clinical trial information by drug and generates the databases by drug 211 and 212 to constitute the clinical trial information DB 210. The database generation unit 232 classifies the real-world data by drug and generates the databases by drug 221 and 222 to constitute the real-world data DB 220.

The number of databases by drug stored in each of the clinical trial information DB 210 and the real-world data DB 220 may be any number according to types of drugs.

Next, the factor identification processing unit 240 is described. The factor identification processing unit 240 of the present embodiment has an input acceptance unit 241, a common factor extraction unit 242, an average value calculation unit 243, a threshold setting unit 244, a division unit 245, an objective function calculation unit 246, a similarity determination unit 247, a difference calculation unit 248, a factor information generation unit 249, a candidate factor identification unit 250, a patient identification unit 251, and an output unit 252.

The input acceptance unit 241 accepts an input to the information processing device 200. For example, the input acceptance unit 241 accepts search instructions of the existing drug and the factor that are input in the terminal device 300 and the like.

The common factor extraction unit 242 extracts a common factor from the clinical trial information and the real-world data corresponding to the existing drug accepted by the input acceptance unit 241 in the clinical trial information DB 210 and the real-world data DB 220.

The average value calculation unit 243 calculates an average value of values of the extracted common factor. The threshold setting unit 244 sets a threshold for the common factor.

The division unit 245 divides a first patient group corresponding to the clinical trial information and a second patient group corresponding to the real-world data according to the threshold.

The first patient group corresponding to the clinical trial information is a patient group having received a clinical trial using the existing drug accepted by the input acceptance unit 241, before this drug is approved. For example, the first patient group refers to a plurality of patients identified by patient identifiers (IDs) included in the clinical trial information for each patient corresponding to the existing drug accepted by the input acceptance unit 241.

The second patient group corresponding to the real-world data is a patient group having received a therapy using the existing drug accepted by the input acceptance unit 241. For example, the second patient group refers to a plurality of patients identified by patient IDs included in the real-world data for each patient corresponding to the existing drug accepted by the input acceptance unit 241.

The objective function calculation unit 246 calculates an objective function for each of new patient groups divided by the division unit 245. The similarity determination unit 247 determines whether behaviors of the divided patient groups are similar to each other based on the values of the objective fu nctions.

The difference calculation unit 248 calculates a difference of the objective functions calculated by the objective function calculation unit 246. For example, the difference calculation unit 248 calculates a difference of the objective functions of two patient groups where it is determined that the behaviors of two patient groups are not similar to each other.

The factor information generation unit 249 generates information indicating a factor related to efficacy of the existing drug accepted by the input acceptance unit 241 among the common factors. This factor information also includes information indicating a factor related to new efficacy of the existing drug. Therefore, the factor information generation unit 249 is an example of a generation unit that generates information indicating a candidate of the factor related to new efficacy of the existing drug.

The new efficacy according to the present embodiment refers to efficacy other than the existing efficacy already known as the efficacy of the existing drug.

The candidate factor identification unit 250 identifies the candidate of the factor related to the new efficacy of the existing drug based on the information generated by the factor information generation unit 249. For example, the candidate factor identification unit 250 identifies a common factor with which a difference of objective functions is maximized among common factors as the candidate of the factor related to the new efficacy of the existing drug.

The patient identification unit 251 identifies patients who satisfy a condition for participating in a clinical trial for evaluating the new efficacy of the existing drug, which is related to the factor identified by the candidate factor identification unit 250.

The output unit 252 outputs the information generated by the factor information generation unit 249, the factor identified by the candidate factor identification unit 250, the information regarding the patients identified by the patient identification unit 251, and the like to the terminal device 300.

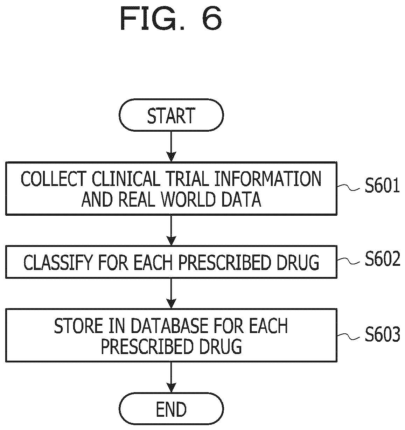

Next, an operation of the information processing device 200 is described with reference to FIGs. 6 to 8. FIG. 6 is a first flowchart describing processing of the information processing device. FIG. 6 illustrates processing by the preprocessing unit 230.

In the preprocessing unit 230 of the information processing device 200 of the present embodiment, the medical information collection unit 231 collects clinical trial information and real-world data (step S601). The collection source of the clinical trial information and the real-world data may be, for example, an electronic medical record system communicable with the information processing system 100, or the like.

Subsequently, in the preprocessing unit 230, the database generation unit 232 classifies the clinical trial information and the real-world data of each patient for each drug prescribed for the patient (step S602).

For example, the database generation unit 232 classifies, among the collected clinical trial information, clinical trial information having the same value of the item "drug" into the same category. For example, the database generation unit 232 classifies, among the collected real-world data, real-world data having the same value of the item "drug" into the same category.

Subsequently, the database generation unit 232 stores the classified clinical trial information and the classified real-world data in the database by drug (step S603), and ends the preprocessing.

According to the present embodiment, in step S602, clinical trial information may be classified for each clinical trial information having the same value of the item "drug" and the same value of the item "dose", and real-world data may be classified for each real-world data having the same value of the item "drug" and the same value of the item "dose".

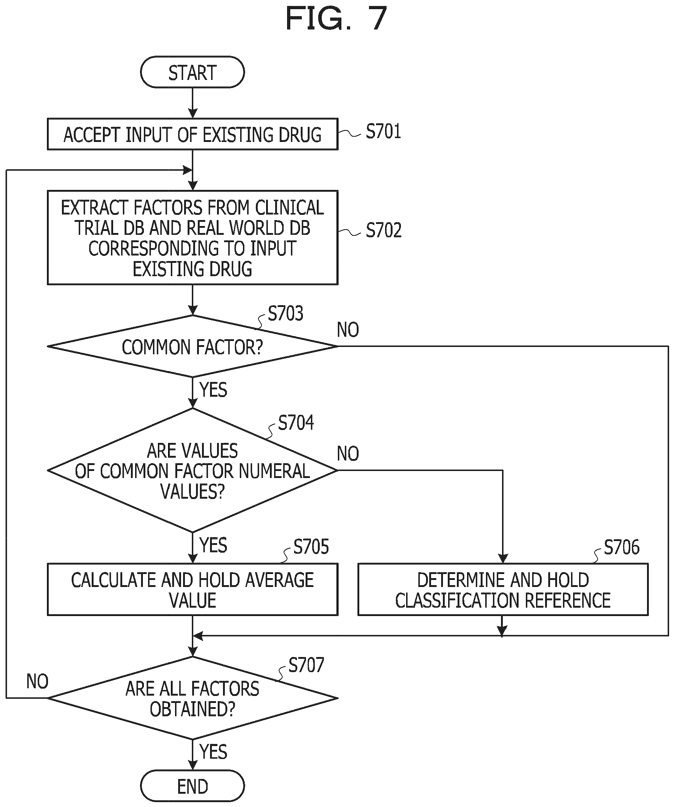

FIG. 7 is a second flowchart describing the processing of the information processing device. FIG. 7 illustrates processing for calculating, by the factor identification processing unit 240, a provisional threshold for dividing the first patient group and the second patient group for each common factor.

In the factor identification processing unit 240 of the information processing device 200, the input acceptance unit 241 accepts an input of an existing drug from the terminal device 300 (step S701).

Subsequently, the common factor extraction unit 242 of the factor identification processing unit 240 extracts factors from the database by drug corresponding to the input existing drug in the clinical trial information DB 210 and the database by drug corresponding to the input existing drug in the real-world data DB 220 (step S702).

For example, the common factor extraction unit 242 extracts factors associated with the item "factor type" from each of the clinical trial information stored in the database by drug corresponding to the input existing drug in the clinical trial information DB 210 and the real-world data stored in the database by drug corresponding to the input existing drug in the real-world data DB 220.

Subsequently, the common factor extraction unit 242 determines whether the factor extracted from the clinical trial information and the factor extracted from the real-world data are common (step S703). For example, the common factor extraction unit 242 determines whether the factor extracted from the clinical trial information is matched with the factor extracted from the real-world data in step S702.

In step S703, when the factors are not common, the factor identification processing unit 240 proceeds to step S706 described below.

In step S703, when the factors are common, the average value calculation unit 243 determines whether values of the common factor are numeric values (step S704). Cases where the values of the common factor are not numeric values include, for example, a case where the common factor is "sex", "race", or the like.

In step S704, when the values of the common factor are numeric values, the average value calculation unit 243 of the factor identification processing unit 240 calculates an average value of the values of the common factor using the values of the common factor in the clinical trial information corresponding to the input existing drug and the values of the common factor in the real-world data corresponding to the input existing drug. The factor information generation unit 249 of the factor identification processing unit 240 holds the common factor and the average value in association with each other (step S705), and proceeds to step S707 described below.

The average value calculated herein is used as a provisional threshold of the common factor for dividing the first patient group and the second patient group.

In step S704, when the values of the common factor are not numeric values, the average value calculation unit 243 determines a value of the common factor which is set as a reference for dividing the first patient group and the second patient group. The factor information generation unit 249 of the factor identification processing unit 240 holds the common factor and the value of the common factor set as the reference in association with each other (step S706).

The value set as the reference for dividing the patient groups may be, for example, "male", "female", "Mongoloid", "Caucasian", or the like.

Following step S705 or step S706, the factor identification processing unit 240 determines whether all factors included in the clinical trial information and the real-world data corresponding to the input existing drug are processed (step S707).

In step S707, when any of the factors are not processed, the factor identification processing unit 240 returns to step S702. In step S707, when all the factors are processed, the factor identification processing unit 240 ends the processing for calculating the provisional threshold.

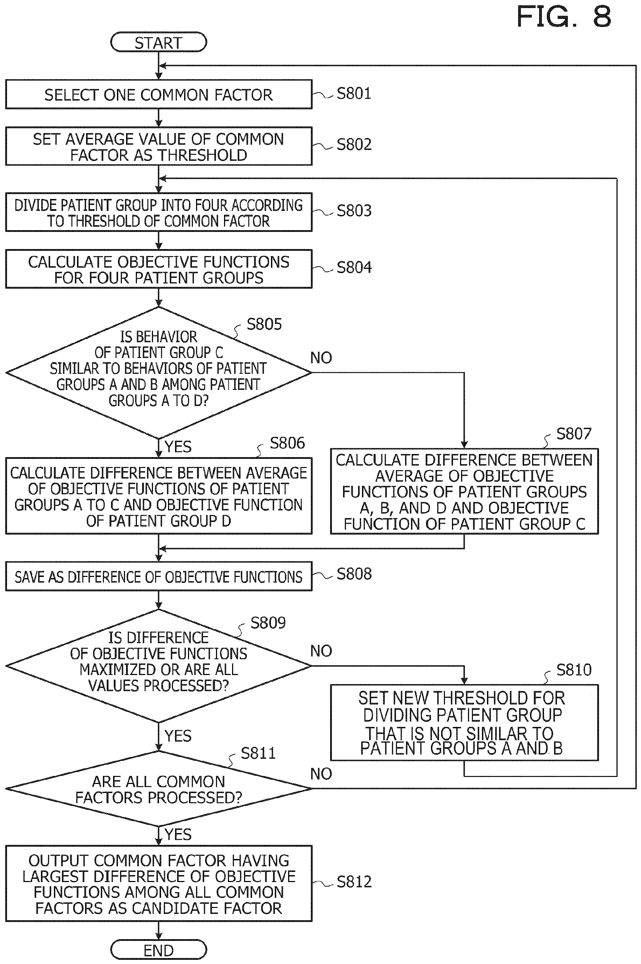

FIG. 8 is a third flowchart describing the processing of the information processing device. FIG. 8 illustrates processing for outputting a factor related to new efficacy of the input existing drug.

In the information processing device 200 of the present embodiment, the threshold setting unit 244 of the factor identification processing unit 240 selects one of held common factors (step S801).

Subsequently, the threshold setting unit 244 sets, as a threshold, an average value associated with the selected common factor (step S802). At this time, when the value associated with the common factor is the reference for the division, this reference may be set as the threshold.

Subsequently, the division unit 245 of the factor identification processing unit 240 divides the first patient group into two new patient groups and divides the second patient group into two new patient groups according to the threshold of the common factor (step S803). The division unit 245 divides the first patient group into a patient group where the value of the common factor is below the threshold (hereinafter, a patient group A), and a patient group where the value of the common factor is equal to or higher than the threshold (hereinafter, a patient group B). The division unit 245 divides the second patient group into a patient group where the value of the common factor is below the threshold (hereinafter, a patient group C), and a patient group where the value of the common factor is equal to or higher than the threshold (hereinafter, a patient group D). For example, the first patient group and the second patient group are divided into the four patient groups in total.

Subsequently, the objective function calculation unit 246 of the factor identification processing unit 240 calculates an objective function for each of the divided four patient groups based on medical information of each of the divided four patient groups (step S804).

For example, the objective function calculation unit 246 calculates an objective function regarding the patient group A based on the clinical trial information corresponding to the patient group A, and calculates an objective function regarding the patient group B based on the clinical trial information corresponding to the patient group B. The objective function calculation unit 246 calculates an objective function regarding the patient group C based on the real-world data corresponding to the patient group C, and calculates an objective function regarding the patient group D based on the real-world data corresponding to the patient group D. The objective function is described later in detail.

Subsequently, the similarity determination unit 247 of the factor identification processing unit 240 determines whether, among the patient group A to the patient group D, a behavior of the patient group C is similar to a behavior of each of the patient group A and the patient group B (step S805). For example, the similarity determination unit 247 determines whether, in the patient group A to the patient group D, as compared with a behavior of the patient group D, the behavior of the patient group C is more similar to the behavior of each of the patient group A and the patient group B.

In this manner, according to the present embodiment, when the first patient group having received the clinical trial of the existing drug and the second patient group having received the therapy using the existing drug are stratified using a certain common factor, it is determined whether a patient group exhibiting a behavior different from that of the first patient group exists, in the second patient group.

In step S805, when a behavior of the patient group C is similar to behaviors of the patient group A and the patient group B, the difference calculation unit 248 of the factor identification processing unit 240 calculates an average of respective objective functions of the patient group A, the patient group B, and the patient group C, and calculates a difference between this average and an objective function of the patient group D (step S806), and proceeds to step S808 described below.

In step S805, when the behavior of the patient group C is not similar to the behaviors of the patient group A and the patient group B, for example, when the behavior of the patient group D is more similar to the behaviors of the patient group A and the patient group B, the difference calculation unit 248 calculates an average of respective objective functions of the patient group A, the patient group B, and the patient group D, and calculates a difference between this average and an objective function of the patient group C (step S807), and proceeds to step S808 described below.

Subsequently, the factor information generation unit 249 holds the difference calculated in step S806 or step S807 as a difference of the objective functions (step S808).

Subsequently, the factor identification processing unit 240 determines whether the difference of the objective functions is maximized or whether the division is performed by all the values of the common factor selected in step S801 (step S809).

For example, the factor identification processing unit 240 determines whether the difference of the objective functions is maximized when the values of the common factor are numeric values, and determines whether the processing up to step S808 is executed by all the values of the common factor when the values of the common factor are not numeric values.

In step S809, when the difference of the objective functions is not maximized or the division is not performed by any value of the common factor selected in step S801, the threshold setting unit 244 of the factor identification processing unit 240 sets a new threshold or a new value of the common factor for dividing patient groups that are not similar to the patient group A and the patient group B (step S810), and returns to step S803.

In step S809, when the difference of the objective functions is maximized or the division is performed by all the values of the common factor selected in step S801, the factor identification processing unit 240 determines whether processing up to step S810 is executed for all the common factors (step S811) .

In step S811, when the processing is not performed for any of the common factors, the factor identification processing unit 240 returns to step S801.

In step S811, when the processing is performed for all the common factors, the candidate factor identification unit 250 of the factor identification processing unit 240 identifies the common factor having the largest difference of the objective functions as a candidate of the factor related to the new efficacy of the existing drug based on the information held by the factor information generation unit 249. The output unit 252 of the factor identification processing unit 240 outputs the identified result to the terminal device 300 (step S812), and ends the processing.

In this manner, according to the present embodiment, by identifying and presenting the candidate of the factor related to the new efficacy of the existing drug to the terminal device 300, for example, a pharmaceutical company attempting drug repositioning may be notified of the factor having a high probability of being related to the new efficacy. Therefore, according to the present embodiment, the search for the factor which has been performed in a trial and error manner may be facilitated.

According to the present embodiment, information in which each common factor is associated with the difference of the objective functions that is calculated for each common factor may be output as information indicating a candidate of the factor related to the new efficacy of the existing drug.

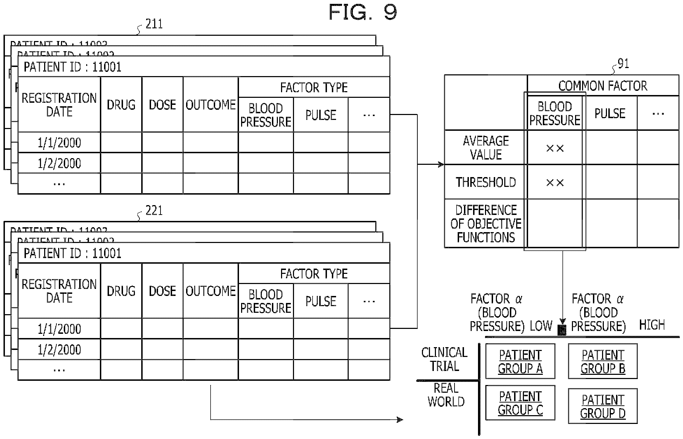

Hereinafter, with reference to FIGs. 9 to 12, the processing of the factor identification processing unit 240 of the present embodiment is further described.

FIG. 9 is a diagram for describing setting of a threshold for a common factor. In FIG. 9, the database by drug 211 is extracted as the clinical trial information corresponding to the input existing drug, and the database by drug 221 is extracted as the real-world data corresponding to the input existing drug.

The common factor extraction unit 242 of the factor identification processing unit 240 extracts the factor "blood pressure" from the factor type of the database by drug 211, and also extracts the factor "blood pressure" from the factor type of the database by drug 221. In the example of FIG. 9, the factor "blood pressure" is a factor common in the clinical trial information and the real-world data, and is therefore set as a common factor.

When "blood pressure" is set as a common factor, values of the common factor are numeric values. Therefore, the average value calculation unit 243 of the factor identification processing unit 240 calculates an average value of all values of "blood pressure" included in the factor type of the database by drug 211 and all values of "blood pressure" included in the factor type of the database by drug 221.

The factor information generation unit 249 of the factor identification processing unit 240 generates and holds information 91 in which "blood pressure" set as the common factor is associated with the calculated average value.

Subsequently, the threshold setting unit 244 sets this average value as a threshold of the common factor "blood pressure". When the threshold is set, the factor information generation unit 249 holds the common factor "blood pressure", the average value of "blood pressure", and the threshold of "blood pressure" in the information 91 in association with each other.

The division unit 245 of the factor identification processing unit 240 divides the first patient group into the patient group A and the patient group B and divides the second patient group into the patient group C and the patient group D by this threshold.

According to the present embodiment, one threshold of the common factor is set to divide the first patient group into two and divide the second patient group into two, but the configuration is not limited to this. According to the present embodiment, for example, two thresholds of the common factor may be set to divide the first patient group into three and divide the second patient group into three.

In this manner, according to the present embodiment, a plurality of thresholds may be set for the common factor, and the number of divisions for the first patient group and the second patient group may be determined by the number of thresholds.

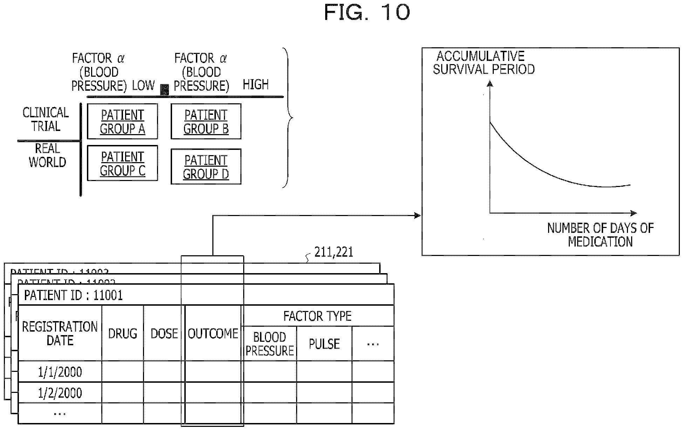

FIG. 10 is a diagram for describing an objective function. The objective function calculation unit 246 of the present embodiment performs survival time analysis based on clinical trial information regarding the patient group A and the patient group B, and obtains respective objective functions of the patient group A and the patient group B. The objective function calculation unit 246 performs survival time analysis based on real-world data regarding the patient group C and the patient group D, and obtains respective objective functions of the patient group C and the patient group D.

According to the present embodiment, a function for defining a relationship between the number of days of medication and an accumulative survival period is set as an objective function. The accumulative survival period of the present embodiment is obtained by the following expression.

Accumulative survival period = (Population parameter of patient group - Number of fully recovered patients of patient group)/(Population parameter of patient group)

According to the present embodiment, a value of the number of fully recovered patients of the patient group is the number of patients where a value of the item "outcome" in each of the clinical trial information and the real-world data is set as "fully recovered".

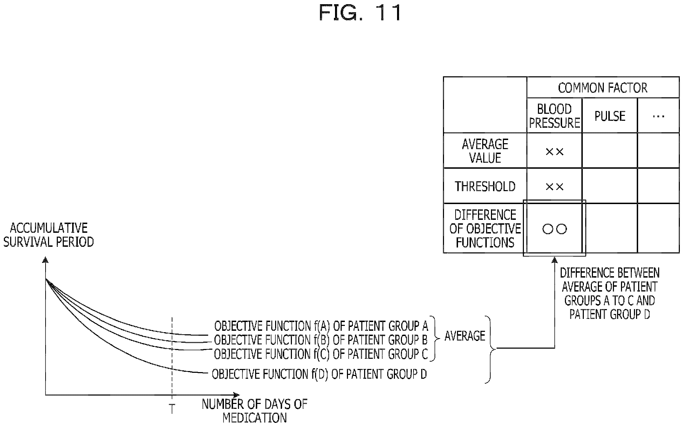

A survival period is a period until the death, a period until the relapse, or the like. The death in this case means an end of a therapy (including full recovery/termination). Next, with reference to FIG. 11, calculation of a difference of objective functions is described. FIG. 11 is a diagram for describing a difference of objective functions.

FIG. 11 illustrates objective functions of the patient groups A to D. In the following description, an objective function of the patient group A is referred to as an objective function f(A), an objective function of the patient group B is referred to as an objective function f(B), an objective function of the patient group C is referred to as an objective function f(C), and an objective function of the patient group D is referred to as an objective function f(D).

According to the present embodiment, the objective function f(A) and the objective function f(B) obtained from the clinical trial information serving as the existing information are set as references.

The similarity determination unit 247 of the present embodiment determines which objective function out of the objective function f(C) and the objective function f(D) obtained from the real-world data is similar to the objective function f(A) and the objective function f(B).

In the example of FIG. 11, a curve representing the objective function f(C) is more similar to a curve representing each of the objective function f(A) and the objective function f(B) than a curve representing the objective function f(D). Therefore, the similarity determination unit 247 determines that the objective function f(C) is determined as an objective function similar to the objective function f(A) and the objective function f(B).

Next, the difference calculation unit 248 of the factor identification processing unit 240 calculates an average of the accumulative survival periods respectively indicated by the three similar objective function f(A), objective function f(B), and objective function f(C). The difference calculation unit 248 calculates a difference between this average and the objective function f(D).

For example, the difference calculation unit 248 calculates a difference between the average value of the respective accumulative survival periods at a time point T in the objective function f(C), the objective function f(A), and the objective function f(B), and a value of the accumulative survival period at the time point T in the objective function f(D) as a difference between the objective functions f(A), f(B), and f(C) and the objective function f(D).

The difference between the objective functions f(A), f(B), and f(C) and the objective function f(D) is not limited to the aforementioned value.

For example, the difference calculation unit 248 may calculate a difference between an average value of inclinations of the respective curves representing the objective functions f(A), f(B), and f(C) at the time point T and an inclination of the curve representing the objective function f(D) as the difference between the objective functions f(A), f(B), and f(C) and the objective function f(D).

For example, the difference calculation unit 248 may calculate a difference between an average value of the areas of figures defined by a vertical axis, a horizontal axis, and the respective curves representing the objective functions f(A), f(B), and f(C) at the time point T and the area of a figure defined by the vertical axis, the horizontal axis, and the curve representing the objective function f(D) as the difference between the objective functions f(A), f(B), and f(C) and the objective function f(D).

For example, the objective function calculation unit 246 may set the objective functions f(A), f(B), f(C), and f(D) as functions indicating relationships between the number of days of medication and survival rates.

In this case, the difference calculation unit 248 may calculate a difference between an average of the numbers of days of medication when the survival rate is 0.5 in the objective functions f(A), f(B), and f(C) and the number of days of medication when the survival rate is 0.5 in the objective function f(D) as the difference between the objective functions f(A), f(B), and f(C) and the objective function f(D).

The difference calculation unit 248 may calculate a difference between an average of inclinations of the respective objective functions when the survival rate is 0.5 in the objective functions f(A), f(B), and f(C) and an inclination of the objective function when the survival rate is 0.5 in the objective function f(D) as the difference between the objective functions f(A), f(B), and f(C) and the objective function f(D).

The difference between the objective functions f(A), f(B), and f(C) and the objective function f(D) is simply referred to as a difference of objective functions in some cases.

According to the present embodiment, when the difference of the objective functions is calculated by the difference calculation unit 248, the factor information generation unit 249 holds the common factor "blood pressure", the average value of "blood pressure", the threshold of "blood pressure", and the difference of the objective functions in the information 91 in association with each other.

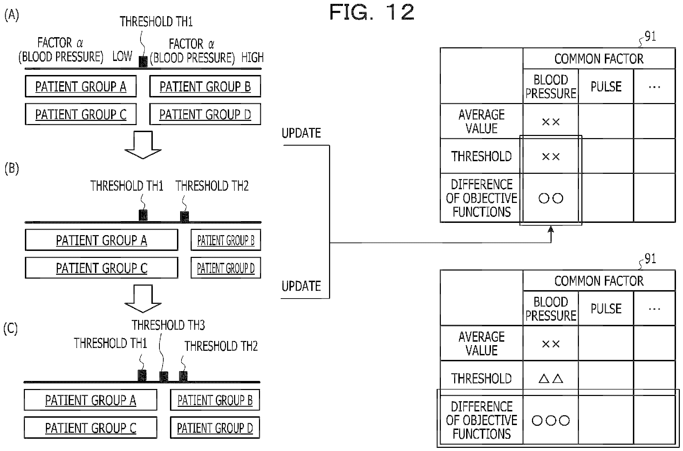

Next, with reference to FIG. 12, resetting of the threshold for the common factor is described. FIG. 12 is a diagram for describing resetting of the threshold for the common factor.

In FIG. 12, (A) illustrates a state where an average value is set as a threshold TH1 for the factor "blood pressure". In FIG. 12, (B) illustrates a state where a new threshold TH2 is set for the factor "blood pressure". In FIG. 12, (C) illustrates a state where a new threshold TH3 is set for the factor "blood pressure".

According to the present embodiment, when the first patient group and the second patient group are divided while the average value of the factor is set as the threshold, for the patient group D where it is determined that the behavior is not similar to those of the patient groups A, B, and C, a threshold for further dividing the patient group D is set again. According to the present embodiment, a threshold at which the difference of the objective functions is maximized is found.

The threshold setting unit 244 of the present embodiment sets again the threshold TH1 that is set in (A) of FIG. 12, as the threshold TH2. The threshold TH2 is set such that the patient group D is divided into two.

For example, the threshold TH2 may be set such that a range from a maximum value of the factor "blood pressure" to the threshold TH1 is divided into two.

According to the present embodiment, when the threshold TH2 is newly set by the threshold setting unit 244, the division unit 245 divides the first patient group and the second patient group, and the processing in step S804 of FIG. 8 is performed.

When the new threshold TH2 is set, the factor information generation unit 249 updates the value of the threshold associated with the common factor "blood pressure" and the value of the newly calculated difference of the objective functions in the information 91, and holds the information 91 after the update.

Similarly as in (C) of FIG. 12, the threshold TH3 is newly set by the threshold setting unit 244, the division unit 245 divides the first patient group and the second patient group, and the processing in step S804 of FIG. 8 is performed.

The factor identification processing unit 240 of the present embodiment repeats this processing until the difference of the objective functions between the average of the objective functions of the patient groups A, B, and C and the objective function of the patient group D is maximized with regard to the common factor "blood pressure".

For example, in this processing, when the first patient group and the second patient group are stratified using the common factor "blood pressure", a patient group exhibiting a behavior most divergent from the behavior of the first patient group among the patient groups included in the second patient group is identified.

According to the present embodiment, each time the threshold of the common factor is changed, the threshold in the information 91 and the difference of the objective functions are also updated. When the processing by the factor identification processing unit 240 is ended, a state is established where each common factor, the maximum value of the difference of the objective functions, and the threshold at which the difference of the objective functions is maximized are in association with each other in the information 91.

Therefore, it is conceivable that the information 91 is information in which a degree of deviation between the behavior of the first patient group having received the clinical trial using the existing drug and the behavior of the second patient group having received the therapy using the existing drug is associated with each common factor. For example, the degree of the deviation is a difference of objective functions.

This difference of the objective functions indicates a response different from the known response to the administration of the existing drug, and it is conceivable that a common factor having a larger difference of the objective functions has a higher probability to be the factor related to the new efficacy of the existing drug.

According to the present embodiment, when the threshold of the common factor is set as described above, for each common factor, the degree of the deviation when the behavior of any patient group (patient group D) included in the second patient group is most divergent from the behavior of the other patient groups may be obtained.

According to the present embodiment, the candidate factor identification unit 250 identifies a common factor having the largest difference of the objective functions among the respective common factors in the information 91 as the candidate of the factor related to the new efficacy of the existing drug. From this, it is conceivable that the information 91 is information regarding the candidate of the factor related to the new efficacy of the existing drug.

In the following description, the candidate of the factor identified by the candidate factor identification unit 250 is referred to as a factor candidate in some cases.

According to the present embodiment, when a common factor having the largest difference of the objective functions among a plurality of common factors extracted from the clinical trial information and the real-world data is set as the factor candidate, the factor having the largest degree of deviation from the known response to the administration of the existing drug may be identified. Therefore, for example, the factor having the highest probability to be the factor related to the new efficacy of the existing drug may be identified.

According to the present embodiment, when the factor candidate is identified, the patient identification unit 251 refers to the real-world data, and extracts the real-world data matched with the factor candidate.

For example, when the factor candidate is a "blood pressure at 130 mmHg or higher", the patient identification unit 251 refers to the real-world data of the second patient group, and extracts the real-world data for each patient having the "blood pressure at 130 mmHg or higher".

The patient identification unit 251 identifies a patient who is identified by a patient ID included in the extracted real-world data as a patient satisfying a participation condition in a new clinical trial for evaluating whether the candidate factor is related to the new efficacy of the existing drug.

Information of the patient identified by the patient identification unit 251 may be output to the terminal device 300 by the output unit 252. The information of the patient may be the number of patients satisfying the participation condition in the new clinical trial or the like.

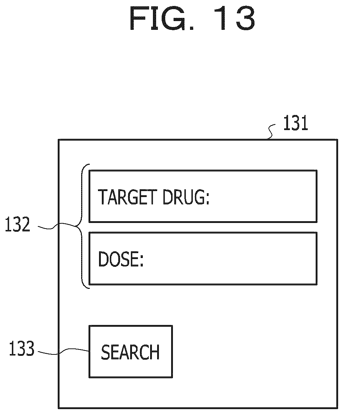

FIG. 13 is a first diagram illustrating a display example. A screen 131 illustrated in FIG. 13 is an example of an input screen of an existing drug displayed in the terminal device 300.

The screen 131 includes an input field 132 and an operation button 133. According to the present embodiment, in the terminal device 300, when an existing drug set as a target of repositioning is input to the input field 132 and the operation button 133 is operated, this existing drug and a factor search instruction are transmitted to the information processing device 200.

The input field 132 includes an input field for inputting a name of the existing drug set as the target of the repositioning and an input field for inputting a dose. According to the present embodiment, for example, when the existing drug and the dose are input to the input field 132, the clinical trial information and the real-world data are extracted by a combination of the existing drug and the dose, a patient group corresponding to the extracted clinical trial information is set as the first patient group, and a patient group corresponding to the extracted real-world data is set as the second patient group.

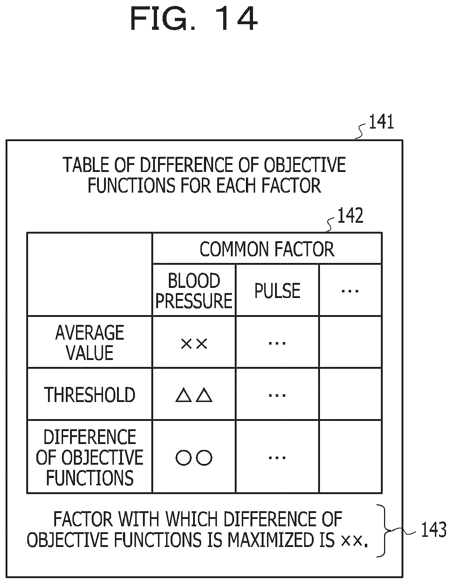

FIG. 14 is a second diagram illustrating a display example. A screen 141 illustrated in FIG. 14 is an example of a processing result by the factor identification processing unit 240, which is displayed in the terminal device 300.

The screen 141 includes display fields 142 and 143. The display field 142 displays the information 91 including the candidate of the factor related to the new efficacy of the existing drug. The display field 143 displays information indicating the candidate factor related to the new efficacy of the existing drug.

According to the present embodiment, since the information including the candidate of the factor related to the new efficacy of the existing drug and the candidate factor related to the new efficacy of the existing drug may be presented as described above, the load from the search for the factor related to the new efficacy in the drug repositioning may be alleviated, and the drug repositioning may be assisted.

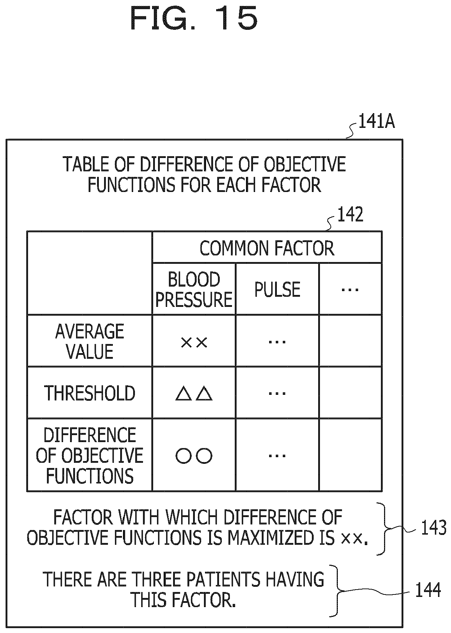

FIG. 15 is a third diagram illustrating a display example. A screen 141A illustrated in FIG. 15 includes display fields 142, 143, and 144.

The display field 144 displays the number of patients including the candidate factor in the real-world data. The display field 144 may display information indicating a medical institution where the patient including the candidate factor in the real-world data has received a consultation or the like.

In this manner, according to the present embodiment, since the information including the candidate of the factor related to the new efficacy of the existing drug and the factor identified by the candidate factor related to the new efficacy of the existing drug may be presented, the load from the search for the factor related to the new efficacy in the drug repositioning may be alleviated, and the drug repositioning may be assisted.

The embodiments are not limited to the specifically disclosed embodiments, and various modifications and changes may be made without departing from the scope of the appended claims.

## Claims

1. An information processing method, comprising:
dividing by a computer, for each of a plurality of factors common in clinical trial information of a first patient group and real-world data of second patient group, each of the first patient group and the second patient group into new patient groups by a predetermined value; and
generating information regarding a candidate of a factor related to new efficacy of an existing drug for each of the plurality of factors based on a behavior of a predetermined patient group related to the second patient group among the new patient groups and behaviors of other patient groups among the new patient groups.

2. The information processing method according to claim 1, further comprising:
identifying, among the plurality of factors, a factor which indicates a state where the behavior of the predetermined patient group is different from the behaviors of the other patient groups, as the candidate of the factor related to the new efficacy of the existing drug.

3. The information processing method according to claim 1 or 2, further comprising:
setting, as the predetermined value, a value at which a difference between an objective function of the predetermined patient group and an average of objective functions of the other patient groups is maximized among objective functions generated for the respective new patient groups.

4. The information processing method according to claim 3, wherein
the information generated for each of the plurality of factors includes the difference between the objective function of the predetermined patient group and the average of the objective functions of the other patient groups, and
the method further comprises:
identifying a factor having a largest difference among the plurality of factors as the candidate of the factor related to the new efficacy of the existing drug.

5. The information processing method according to any one of claims 1 to 4, further comprising:
outputting information including the candidate of the factor related to the new efficacy of the existing drug.

6. The information processing method according to any one of claims 2 to 5, further comprising:
outputting information indicating the factor identified as the candidate of the factor related to the new efficacy of the existing drug.

7. The information processing method according to any one of claims 2 to 6, further comprising:
outputting information regarding a patient satisfying a condition for a participation in a new clinical trial related to an evaluation of the new efficacy of the existing drug based on the factor identified as the candidate of the factor related to the new efficacy of the existing drug and the real-world data.

8. An information processing program that causes a computer to execute a process, the process comprising:
dividing, for each of a plurality of factors common in clinical trial information of a first patient group and real-world data of second patient group, each of the first patient group and the second patient group into new patient groups by a predetermined value; and
generating information regarding a candidate of a factor related to new efficacy of an existing drug for each of the plurality of factors based on a behavior of a predetermined patient group related to the second patient group among the new patient groups and behaviors of other patient groups among the new patient groups.

9. An information processing device, comprising:
a division unit that divides, for each of a plurality of factors common in clinical trial information of a first patient group and real-world data of second patient group, each of the first patient group and the second patient group into new patient groups by a predetermined value; and
a factor information generation unit that generates information regarding a candidate of a factor related to new efficacy of an existing drug for each of the plurality of factors based on a behavior of a predetermined patient group related to the second patient group among the new patient groups and behaviors of other patient groups among the new patient groups.
